# EUROPÄISCHE PATENTSCHRIFT

(11) **EP 3 289 781 B1**
(45) Veröffentlichungstag und Bekanntmachung des Hinweises auf die Patenterteilung: **26.06.2019**
(21) Anmeldenummer: 16719381.2
(22) Anmeldetag: 27.04.2016
(51) Int. Cl.: H04R 25/00

(54) **ULTRASCHALLPULSE AUSSENDENDE HÖRPROTHESE**
HEARING AID EMITTING ULTRASONIC PULSES
APPAREIL AUDITIF ÉMETTANT DES IMPULSIONS ULTRASONIQUES

(30) Priorität: 28.04.2015 DE 102015106560
(43) Veröffentlichungstag der Anmeldung: 07.03.2018
(73) Patentinhaber: Audisense GmbH, 23560 Lübeck (DE)
(72) Erfinder: HERRMANN, Vera, 23560 Lübeck (DE)
(74) Vertreter: von dem Borne, Andreas
(86) Internationale Anmeldenummer: PCT/EP2016/059364
(87) Internationale Veröffentlichungsnummer: WO 2016/174065

(56) Entgegenhaltungen:
- US-A1- 2005 201 574
- US-A1- 2014 355 800
- LENHARDT M L: "Ultrasonic hearing in humans: applications for tinnitus treatment", INTERNATIONAL TINNITUS JOURNAL, BAD KISSINGEN, Bd. 9, Nr. 2, 1. Januar 2003 (2003-01-01), Seiten 69-75, XP008097998, ISSN: 0946-5448

## Beschreibung

Die Erfindung betrifft eine Hörprothese und ein Verfahren zum Ausgleich von Hörverlusten oder Hörstörungen mit einer solchen Hörprothese.

Vor dem Hintergrund eines sehr großen Bevölkerungsanteils mit Hörschädigungen und aufgrund der starken Beeinträchtigung der Betroffenen besteht in der Praxis ein besonders großes Interesse an Hörprothesen bzw. Hörgeräten. Bei intaktem Hörvermögen sammelt die Ohrmuschel die Schallwellen aus der Umgebung und leitet diese durch den Gehörgang zum Trommelfell, welches zum Schwingen angeregt wird. Diese Schwingungen werden über das Mittelohr auf eine Flüssigkeit im Innenohr bzw. in der Gehörschnecke (Cochlea) übertragen. Durch Bewegung der Flüssigkeit werden feine Haarzellen innerhalb der Cochlea ausgelenkt und dabei werden (bioelektrische) Nervenimpulse ausgelöst, die über den Hörnerv an das Gehirn weitergeleitet werden, so dass im Gehör die Hörwahrnehmung entsteht.

Konventionelle Hörgeräte arbeiten elektroakustisch und folglich mit akustischer Verstärkung. Dieses Prinzip funktioniert jedoch dann nicht mehr, wenn in der Cochlea keine bioelektrischen Signale mehr erzeugt werden können.

In solchen Fällen können gegebenenfalls sogenannte Cochlea-Implantate helfen. Diese wandeln zunächst Schall in elektrische Impulse um, und diese stimulieren durch direkt in die Cochlea implantierte Elektroden die Nerven im Innenohr bzw. den Hörnerv. Auf diese Weise können Sprache und Geräusche wieder wahrgenommen werden. Das Funktionsprinzip solcher Cochlea-Implantate beruht auf der sogenannten "Tonotopie" der Cochlea. In der Cochlea werden die von außen kommenden mechanischen Schwingungen in neuronale Impulse umgewandelt, und zwar anatomisch geordnet nach Frequenz. Verschiedene räumlich verteilte Bereiche innerhalb der Cochlea sind für die Wahrnehmung unterschiedlicher Frequenzen verantwortlich, hohe Frequenzen werden am äußeren Ende verarbeitet, tiefe Frequenzen am inneren Ende. In der Hörbahn im Gehör wird diese anatomische Sortierung nach Frequenz bis in verschiedene Bereiche des Gehirns beibehalten. In der Cochlea ist das System der Haarzellen folglich so geordnet, dass jede hörbare akustische Frequenz ihren spezifischen Ort der maximalen Empfindlichkeit hat. Dadurch ist jedem Ort auf der sogenannten Basilarmembran eine bestimmte Frequenz zugeordnet. In der Cochlea erfolgt daher eine "Frequenz-Orts-Abbildung", die auch als Tonotopie bezeichnet wird. Diese Funktionsweise der Cochlea wird bei Cochlea-Implantaten ausgenutzt, denn durch die räumlich in der Cochlea verteilten Elektroden des Implantates können gezielt elektrische Impulse erzeugt werden, die eine bestimmte Tonfrequenz repräsentieren. Nachteilig ist dabei, dass eine derartige Hörprothese invasiv als Implantat in die Cochlea integriert werden muss. Dieses ist aufwendig und für den Patienten belastend. Korrekturen lassen sich nicht ohne weiteres vornehmen. Im Übrigen werden aufgrund der Leitfähigkeit des das Implantat umgebenden Gewebes durch die elektrischen Impulse unerwünscht mehrere Nerven angeregt, die für das Hören unterschiedlicher Frequenzen verantwortlich sind. Damit wird die Selektivität des Hörens, die innerhalb der Cochlea erzeugt werden soll, stark reduziert.

Alternativ wurden daher bereits modifizierte Cochlea-Implantate vorgeschlagen, die auf dem Prinzip der Lichtstimulation und optogenetischer Aktivierung beruhen. Dabei wird statt der Elektroden ein Array von Laserdioden in die Cochlea implantiert. Mit Hilfe der Laserstrahlen lässt sich der gewünschte Ort (am Hörnerv) sehr gezielt stimulieren. Die Selektivität des Hörens wird dadurch gegenüber herkömmlichen Cochlea-Implantaten deutlich erhöht. Um allerdings die Nervenzellen der Hörschnecke mit Licht aktivieren zu können, müssen die Nervenzellen zunächst künstlich auf Licht sensibilisiert werden. All diese Maßnahmen sind sehr aufwendig und ebenfalls nur invasiv möglich.

Sofern solche Cochlea-Implantate aufgrund des Grades der Schädigung des Hörsystems nicht einsetzbar sind, können gegebenenfalls auditorische Hirnstamm-Implantate (ABI) zum Einsatz kommen. Das Prinzip entspricht dem der Cochlea-Implantate, wobei mit Elektroden nicht Bereiche innerhalb der Hörschnecke gereizt werden, sondern eine Stufe weiter in der Hörbahn am Nucleus Cochlearis. Solche Systeme können dann zum Einsatz kommen, wenn z. B. aufgrund von Krankheiten die Cochlea oder gar der Hörnerv beschädigt sind oder gar entfernt werden müssen. Der Einsatz von Hirnstamm-Implantaten ist jedoch nur mit hochspezialisierten Maßnahmen möglich. Grundsätzlich besteht folglich bei Patienten, bei denen klassische Cochlea-Implantate nicht funktionieren, das Bedürfnis, an anderen Stationen der Hörbahn anzusetzen. Dazu gibt es auch Überlegungen, elektrische Stimulationen am Nucleus Cochlearis im Hirnstamm oder am Colliculus Inferior vorzunehmen. Stets wären aufwendige invasive Maßnahmen erforderlich.

Es besteht folglich das große Bedürfnis, eine Hörprothese zu schaffen, die bei verschiedensten Arten von Hörverlusten oder Hörschädigungen/-störungen ohne operative Maßnahmen und folglich nichtinvasiv eingesetzt werden kann. Der Erfindung liegt daher die Aufgabe zugrunde, eine solche Hörprothese zu schaffen.

Zur Lösung dieser Aufgabe lehrt die Erfindung eine Hörprothese mit zumindest
- einem Schallwandler (z. B. Mikrophon), zur Umwandlung akustischer Signale in elektrische Signale und
- einer Ultraschalleinrichtung, mit welcher eine Vielzahl von fokussierten (bzw. dynamisch fokussierbaren), gepulsten Ultraschallstrahlen (mit einer oder mehreren Ultraschallfrequenzen) erzeugbar sind,
wobei die Ultraschalleinrichtung im Außenohr und/oder außerhalb des Ohres (eines menschlichen Körpers) befestigbar ist und
wobei die Ultraschallstrahlen in Abhängigkeit von den vom Schallwandler erzeugten elektrischen Signalen (transkraniell) auf verschiedene räumlich verteilte Punkte in einem Bereich des Innenohrs oder in einem Bereich der Hörbahn im Gehirn (dynamisch) unter Stimulierung von Nerven fokussierbar sind.

Die Erfindung schlägt folglich eine nichtinvasive Ultraschall-Exo-Hörprothese vor. Dabei geht die Erfindung von der grundsätzlich bekannten Erkenntnis aus, dass Hörverluste auch bei Schädigungen in der Cochlea ausgeglichen werden können, wenn eine räumlich selektive Reizung der Nervenzellen in der Cochlea oder auch in anderen Bereichen der Hörbahn vorgenommen wird. Während jedoch bei herkömmlichen Cochlea-Implantaten eine unmittelbare Reizung mit elektrischen oder optischen Impulsen erfolgt, werden die Nerven bzw. Nervenzellen erfindungsgemäß mit fokussierter Ultraschallstrahlung stimuliert. Dabei wird - ähnlich wie bei herkömmlichen Cochlea-Implantaten - die Tonotopie der Cochlea ausgenutzt. So werden die besonderen anatomischen Eigenschaften ausgenutzt, indem mit Hilfe einer Vielzahl fokussierter Ultraschallstrahlen gezielt räumlich verteilte Bereiche bzw. Nerven stimuliert werden, welche unterschiedliche Schallfrequenzen repräsentieren. Der natürliche Weg des Schalls wird dabei vollständig umgangen. Im Gegensatz zu Cochlea-Implantaten kann die erfindungsgemäße Hörprothese jedoch im Außenohr oder sogar außerhalb des (menschlichen) Ohres platziert und befestigt werden. Es erfolgt eine transkranielle Stimulierung der für die verschiedenen Frequenzbereiche verantwortlichen Nervenzellen mit fokussierter Ultraschallstrahlung.

Dabei geht die Erfindung von der Erkenntnis aus, dass sich Nerven- und Muskelfasern durch (fokussierten) Ultraschall stimulieren lassen. Dieses wurde in einer Vielzahl von Experimenten in der Vergangenheit nachgewiesen. Dabei verursacht Ultraschall mit hoher Energie (>1 W/cm²) neuronale Aktivitäten und zusätzlich thermische Effekte. Im Vordergrund der vorliegenden Überlegungen stehen nur neuronale Stimulationen, die mit Hilfe gepulster niederfrequenter Ultraschallstrahlung mit niedriger Intensität erzeugt werden. Die Wirkung ist folglich auf mechanische Bioeffekte ohne thermische Effekte und insbesondere ohne Gewebebeschädigung beschränkt. Dass sich Nervenzellen und insbesondere auch ein Hörnerv von Lebewesen mit Hilfe gepulster Ultraschallstrahlung stimulieren lassen, wurde experimentell vielfach nachgewiesen (vgl. Leonid R. Gavrilov et al: A STUDY OF RECEPTION WITH THE USE OF FOCUSED ULTRASOUND, BRAIN RESEARCH, 135 (1977) 279-285 und Vincent Colucci et al: FOCUSED ULTRASOUND EFFECTS ON NERVE ACTION POTENTIAL IN VITRO, Ultrasound in Med. & Biol., Vol. 35, No. 10, 1737-1747, 2009 sowie Yusuf Tufail: Transcranial Pulsed Ultrasound Stimulates Intact Brain Circuits, Neuron 66, 681-694, 2010).

Bevorzugt wird mit Ultraschallpulsen mit einer Pulslänge von 0,01 ms bis 1 ms, vorzugsweise 0,05 ms bis 0,5 ms gearbeitet, wobei die Ultraschallfrequenz bevorzugt geringer als 1000 kHz, vorzugsweise geringer als 700 kHz, z. B. 100 bis 900 kHz, besonders bevorzugt 250 bis 700 kHz beträgt. Dabei können alles Strahlen dieselbe Frequenz aufweisen. Die einzelnen Ultraschallpulse können dabei jeweils eine Pulsleistung von 0,01 bis 0,1 W/cm², das heißt 10 mW/cm² bis 100 mW/cm² aufweisen. In dem beschriebenen Frequenzbereich lässt sich ein optimales Verhältnis zwischen transkranieller Transmission einerseits und der Absorption der Ultraschallstrahlung in den Nervenzellen andererseits erreichen. Der Ultraschallreiz/Stimulationsultraschallpuls muss dabei in der Lage sein, die Stimulation zeitgemäß entsprechend einem Zeitverlauf des Aktionspotentials und der Latenzzeit des Hörnervs wiederholt nach dem akustischen Bedarf erfolgreich durchzuführen. Die beschriebene Pulslänge ist deshalb zweckmäßig, weil längere Stimulationen zur Blockierung des Hörnervs führen können. Solche Stimulationspulse können jedoch erfindungsgemäß bei Bedarf durch Blockierung des Hörnervs auch zur Kompensation eines Tinnitus genutzt werden.

Erfindungsgemäß wird folglich eine medizinische Vorrichtung zur Verfügung gestellt, die die Hörverluste bei Schwerhörigkeit bis hin zur vollen Taubheit mittels fokussiertem, gepulstem Ultraschall nicht invasiv ausgleicht und alternativ auch Tinnituserkrankungen "kompensieren" kann. Dabei ist auch von Bedeutung, dass Patienten, bei denen grundsätzlich Hörverluste mit Hilfe eines Cochlea-Implantates ausgeglichen werden könnten, häufig keinerlei Hörerfahrung haben. Diese Tatsache erschwert jeden Test des Cochlea-Implantates im Vorfeld aus Sicht der Psychoakustik. Mit der erfindungsgemäßen nichtinvasiven Ultraschall-Exo-Hörprothese ist es demgegenüber möglich, besonders solche psychoakustischen Effekte durch Test mit Personen mit intaktem Hörvermögen - z. B. in einem schallisolierten Raum - zu simulieren und in dem Sprachprozessor der Hörprothese korrekt abzubilden. Es ist eine mehrkanalige Schallanalyse und gleichzeitig die mehrkanalige Schallerzeugung in der Hörbahn möglich, wie dies durch ein intaktes Ohr realisiert würde. Die erfindungsgemäße Exo-Hörprothese ist folglich nicht nur ohne Operation anwendbar, sondern sie ist auch optimal und individuell an dem Grad des Hörverlustes anpassbar. Die Frequenzselektivität entspricht dem gesunden Gehör und es wird insbesondere auch räumliches Hören ermöglicht. Besonders vorteilhaft ist die Tatsache, dass eine solche Exo-Hörprothese austauschbar ist und unabhängig vom Alter der Personen und folglich bei verschiedenen Generationen einsetzbar ist.

Von besonderer Bedeutung ist erfindungsgemäß die Erzeugung einer Vielzahl von (dynamisch) fokussierten Ultraschallstrahlen, um die räumlich selektive Stimulation der gewünschten Bereiche, z. B. in der Cochlea oder aber am Hörnerv zu erreichen. Zu diesem Zweck kann die Ultraschalleinrichtung neben einem Ultraschalltreiber zumindest ein Ultraschall-Array aufweisen, mit welchem eine Vielzahl von dynamisch fokussierbaren Ultraschallstrahlen erzeugt werden können, die vorzugsweise alle dieselbe Ultraschallfrequenz aufweisen. Die dynamische Fokussierung von Ultraschallstrahlen bzw. Ultraschallwellen ist in der medizinischen Diagnostik mit Hilfe von Ultraschall-Arrays üblich. Die Elemente des Ultraschall-Arrays werden in mehrere Gruppen aufgeteilt, so dass mit der Ultraschalleinrichtung bevorzugt mehr als 10, besonders bevorzugt mehr als 20 dynamisch fokussierbare und ablenkbare Ultraschallstrahlen erzeugt werden. Die Anzahl der Gruppen wird unter Berücksichtigung der realistischen Frequenzselektivität aufgeteilt, so werden z. B. 20 bis 30 Gruppen vorgesehen. Diese Anzahl der Gruppen von Array-Elementen entspricht dann z. B. der Anzahl der Frequenzgruppen in der Cochlea. Die einzelnen Array-Elemente einer Gruppe sind räumlich verteilt in Array angeordnet. Die Energie eines "Einzelstrahls" aus einem einzelnen Element ist insbesondere unter Berücksichtigung der Absorption in dem Gewebe verhältnismäßig klein. Erst in einem kleinen, fein verstellbaren Fokus der Strahlen einer Gruppe an einer bestimmten Position innerhalb der Cochlea (oder auch am Hörnerv) erreicht die Pulsenergie den gewünschten Pegel, um die erforderliche Stimulation durchzuführen. Jede Gruppe von Array-Elementen ist folglich für die Stimulation einer bestimmten Region der Nervenfasern verantwortlich.

Dabei ist eine dynamische Fokussierung und folglich eine ständige Anpassung der Fokuslage vorteilhaft. Das Ultraschall-Array ist zwar starr aufgebaut, so dass jedes Element und auch jede Gruppe eine feste Position (relativ zueinander) besitzt. Es ist jedoch in der Praxis keine absolute Fixierung der Ultraschalleinrichtung bzw. des Arrays an der Haut oder im Außenohr möglich. Da sich die Position der Ultraschalleinrichtung bzw. des Arrays folglich verändern kann, ist eine geeignete Korrektur erforderlich. Dazu schlägt die Erfindung in besonders bevorzugter Weiterbildung vor, dass mit der Ultraschallquelle ein oder mehrere Referenzstrahlen erzeugt werden, welche unabhängig von den mit dem Schallwandler erzeugten Signalen auf ein oder mehrere Referenzpunkte (z. B. Referenzpunkte in oder an der Cochlea) fokussiert werden. Die Position der Ultraschalleinrichtung, z. B. des Ultraschall-Arrays wird folglich während des Betriebes kontinuierlich durch ein oder mehrere Referenzstrahlen geprüft, indem z. B. die Spitze der Cochlea abgetastet wird. Im Zuge einer "Inbetriebnahme" werden zunächst Informationen über Position, Größe und Orientierung der Cochlea durch einen (dreidimensionalen) Ultraschallscan der ganzen Cochlea ermittelt. Auf dieser Basis werden dann die "Zielpunkte" für die räumlich selektive Stimulation mit den einzelnen Ultraschallstrahlen festgelegt. Sofern mit Hilfe der Referenzstrahlen veränderte Abstände und Einstrahlwinkel von einem Steuerungsprogramm festgestellt werden, werden die Fokustiefen und die Einstrahlwinkel der einzelnen Gruppen relativ zu dem Referenzstrahl oder den Referenzstrahlen in Echtzeit korrigiert.

Das Hörgerät weist zusätzlich zu dem Schallwandler (Mikrofon) und dem Ultraschall-Array/Ultralschalltreiber außerdem z. B. einen Analog-DigitalWandler, einen Sprachprozessor und/oder einen Codierer auf. Außerdem ist eine Energieversorgungseinheit vorgesehen.

In dem Hörgerät ist das Steuerungsprogramm gespeichert, welches die Ultraschallstrahlen (z. B. Winkel, Fokus etc.) berechnet und steuert und auch die berechneten Korrekturen durchführt.

Gegenstand der Erfindung ist auch ein Verfahren zum Ausgleich von Hörverlusten oder Hörstörungen mit einer derartigen Hörprothese, bzw. ein Verfahren zum Betrieb bzw. zur Nutzung/Verwendung einer solchen Hörprothese, wobei die Hörprothese im Außenohr und/oder außerhalb des Ohres befestigt wird, wobei die Ultraschallstrahlen in Abhängigkeit von dem vom Schallwandler erzeugten elektrischen Signalen (transkraniell) auf verschiedene räumlich verteilte Punkte in einem Bereich des Innenohrs oder in einem Bereich der Hörbahn im Gehirn fokussiert werden und dadurch verschiedene räumlich verteilte Regionen (von Nerven) in der Hörschnecke oder in einem Bereich der Hörbahn, welche unterschiedliche Schallfrequenzen repräsentieren, stimulieren.

Dabei kann - wie bereits beschrieben - z. B. eine Stimulierung der Nerven innerhalb der Hörschnecke erfolgen. Es sind jedoch auch andere Stimulationsorte möglich, z. B. eine Stimulation direkt am Hörnerv oder auch eine Stimulation des Nucleus Cochlearis sowie des Colliculus Inferior. Entsprechende Positionen können z. B. mittels MRT bestimmt werden. Auch bei diesen Stimulationsorten kann eine Korrektur mit Hilfe von Referenzstrahlen erfolgen. Die Referenzstrahlen können wiederum auf die Cochleaspitze fixiert werden oder es kann ein anderer Fixpunkt im Schädel gewählt werden.

Wie bereits beschrieben, wird zur Erzeugung einer Vielzahl von dynamisch fokussierbaren Ultraschallstrahlen ein Ultraschall-Array verwendet, dessen Array-Elemente in Gruppen aufgeteilt und zusammengefasst sind, welche jeweils einen akustischen Frequenzbereich repräsentieren, wobei durch Überlagerung der in unterschiedlichen Winkeln eintreffenden Strahlen der einzelnen Elemente in einem Punkt eine Stimulation erfolgt. Dabei kann zumindest ein Referenzstrahl unabhängig von dem mit dem Schallwandler erzeugten Signalen auf zumindest einen Referenzpunkt fokussiert werden und dabei kann die Lage der Fokussierungen der übrigen Ultraschallstrahlen in Abhängigkeit von dem Referenzstrahl in Echtzeit dynamisch korrigiert werden.

Der Fokussierung der Ultraschallstrahlen kommt erfindungsgemäß besondere Bedeutung zu. Dieses gelingt z. B. in der beschriebenen Weise mit Hilfe von Ultraschall-Arrays. Dieses Verfahren lässt sich weiter dadurch optimieren, dass für die Stimulation eines bestimmten Bereichs der Nervenzellen nicht lediglich ein fokussierter Ultraschall verwendet wird, sondern zumindest zwei mit Abstand zueinander erzeugte Ultraschallstrahlen, die auf einen gemeinsamen Punkt fokussiert werden. Die einzelnen Fokusvolumina sind lateral schmal, aber axial gegebenenfalls verhältnismäßig lang. Durch die Anwendung von z. B. Ultraschall-Arrays wird es möglich, durch Superposition von mehreren (zumindest zwei) Ultraschallfeldern ein Fokusvolumen mit reduzierter Größe zu erreichen. Durch die Überlagerung erfolgt eine Reduzierung des Fokusvolumens, in welchem der erforderliche Ultraschallpegel für die Stimulation erreicht wird. Eine solche Anordnung ist sehr günstig, da auf diese Weise im umgebenden Gewebe der Ultraschall keine Belastung hervorruft und auch keine zusätzlichen Reaktionen. So wird z. B. ein Rauschen durch ungewünschte Stimulationen oder Inhibitionen nahestehender Nervenfasern vermieden.

Der Einsatz von Ultraschallstrahlung im Zusammenhang mit Hörgeräten oder Hörprothesen ist zwar bereits bekannt. Dabei dient die Ultraschallfrequenz als Trägerfrequenz für Audiosignale, so dass z. B. von Ultraschall induzierte Signale über Hautvibrationen auf den Bereich des Gehörs oder des Gehirns übertragen und dort decodiert werden können (vgl. z. B. US 6 631 197 B1, US 2004/0196998 A1, WO 00/21440 A1 und WO 2005/072168 A2 sowie US 2014/0355800 A1). Demgegenüber steht erfindungsgemäß eine direkte räumlich selektierte Stimulierung von Nerven mit fokussierten Strahlen im Vordergrund, wobei vorzugsweise die verwendeten Ultraschallstrahlen dieselbe Frequenz aufweisen.

Im Folgenden wird die Erfindung anhand einer lediglich ein Ausführungsbeispiel darstellenden Zeichnung näher erläutert. Es zeigen:
- Fig. 1: schematisch stark vereinfacht eine erfindungsgemäße Hörprothese,
- Fig. 2: die Hörprothese nach Fig. 1 bei Positionierung im Außenohr,
- Fig. 3: den Gegenstand nach Fig. 2 einerseits und eine Ausführungsform bei Positionierung außerhalb des Ohres andererseits,
- Fig. 4: den Gegenstand nach Fig. 1 in einer anderen Darstellung,
- Fig. 5: schematisch vereinfacht weitere Einsatzbereiche der erfindungsgemäßen Hörprothese,
- Fig. 6: eine graphische Darstellung der Tonotopie der Cochlea und
- Fig. 7: die Überlagerung mehrerer Ultraschallstrahlen bei einer Ausführungsform z. B. nach Fig. 1.

In den Figuren ist eine erfindungsgemäße Exo-Hörprothese zum Ausgleich von Hörverlusten oder Hörstörungen dargestellt. Diese Hörprothese weist zumindest einen Schallwandler 1 (z. B. ein Mikrofon zur Umwandlung akustischer Signale in elektrische Signale) auf. Außerdem weist die Hörprothese zumindest eine Ultraschalleinrichtung 2 auf, mit welcher eine Vielzahl von fokussierten, gepulsten Ultraschallstrahlen 3 erzeugt werden. Diese Ultraschalleinrichtung wird im Außenohr A oder außerhalb des Ohres befestigt. Die Ultraschallstrahlen 3 werden in Abhängigkeit von den vom Schallwandler 1 erzeugten elektrischen Signalen transkraniell auf verschiedene räumlich verteilte Punkte P (hinter dem Trommelfell T und hinter dem Mittelohr M) in einem Bereich des Innenohrs I oder in einem Bereich der Hörbahn im Gehirn G dynamisch unter Stimulierung von Nerven fokussiert.

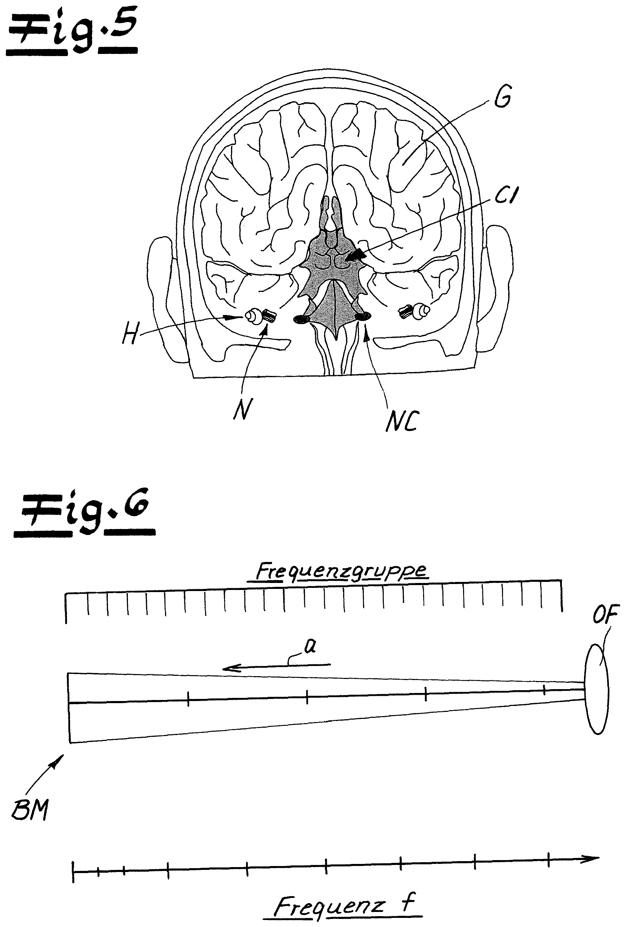

Dabei basiert die erfindungsgemäße Hörprothese auf dem in Fig. 6 illustrierten Zusammenhang zwischen einer räumlichen Anordnung der "ausgerollten Basilarmembran" und der entsprechenden Frequenz. Bei einem gesunden Ohr werden die von außen aufgenommen Schallwellen über das Trommelfell auf das Innenohr I und folglich in die Hörschnecke H (Cochlea) übertragen. Die in der Cochlea H angeordnete Flüssigkeit lenkt dann die Basilarmembran BM, welche die Cochlea H in zwei Kammern teilt, aus. Die Ausbreitungsrichtung a der erzeugten Wanderwelle auf der Basilarmembran und das ovale Fenster OF sind angedeutet. Die Bewegung der Basilarmembran führt zur Bewegung von Haarzellen, wobei das System der Haarzellen anatomisch bedingt so geordnet ist, dass jede hörbare akustische Frequenz ihren spezifischen Ort der maximalen Empfindlichkeit hat. Dieses ist in Fig. 6 angedeutet. Diese Frequenz-Orts-Abbildung wird auch als Tonotopie der Cochlea H bezeichnet. Dieses Prinzip machen sich auch herkömmliche Cochlea-Implantate zu Nutze, indem dort räumlich verteilte Bereiche elektrisch über Elektroden stimuliert werden.

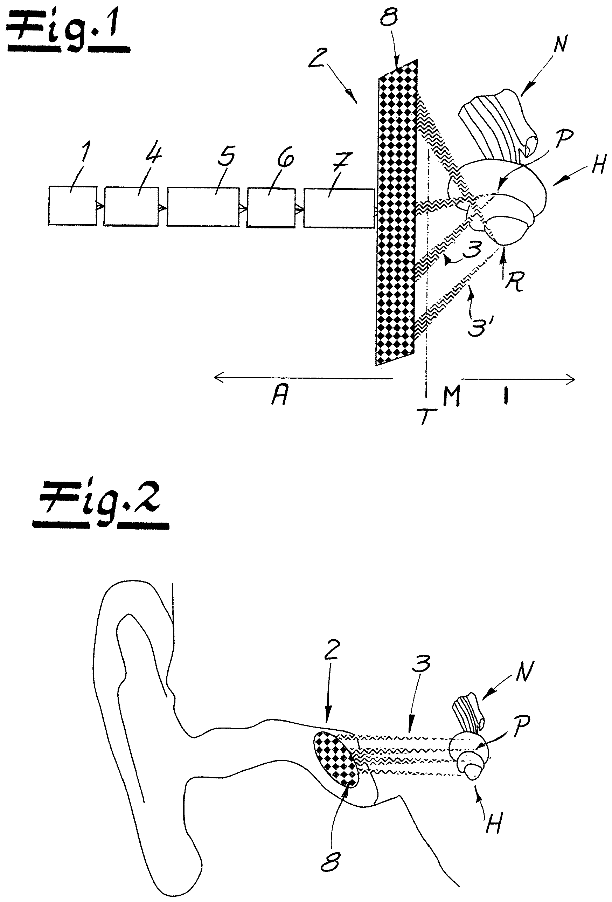

Erfindungsgemäß erfolgt nun eine Stimulation der verschiedenen Bereiche mit Hilfe fokussierter Ultraschallstrahlung 3. Gemäß Fig. 1 werden mit einem Mikrofon 1 Schallwellen in elektrische Impulse umgewandelt und über einen Analog/Digital-Wandler 4 an einen Sprachprozessor 5 übermittelt, welcher diese Signale verarbeitet. Die frequenz- und zeitaufgelösten Amplituden der Ultraschallstrahlen werden mit einem Codierer 6 mehrkanalig codiert, so dass mehrkanalige Steuerungssignale für einen Ultraschalltreiber 7 einer Ultraschalleinrichtung 2 erzeugt werden. Mit Hilfe dieser Steuerungssignale erzeugt die Ultraschalleinrichtung 2 eine Vielzahl von Ultraschallstrahlen 3, die folglich in Abhängig von den vom Schallwandler 1 erzeugten elektrischen Signalen auf verschiedene räumlich verteilte Punkte P in einem Bereich des Innenohrs I oder in einem Bereich der Hörbahn im Gehirn G fokussiert werden, so dass verschiedene räumlich verteilte Regionen von Nerven in der Hörschnecke oder in einem Bereich der Hörbahn stimuliert werden, welche unterschiedliche Schallfrequenzen (gemäß Fig. 6) repräsentieren.

Die Fig. 1 bis 4 zeigen dabei Ausführungsformen, bei denen mit Hilfe der Ultraschallstrahlen 3 Nervenzellen in der Cochlea H stimuliert werden. Jeder einzelne Ultraschallstrahl 3 wird durch entsprechende Steuerungssignale mit einem bestimmten Einstrahlwinkel auf eine bestimmte Tiefe fokussiert.

Dazu weist die Ultraschalleinrichtung 2 im Ausführungsbeispiel ein Ultraschall-Array 8 auf, mit welchem eine Vielzahl von dynamisch fokussierbaren Ultraschallstrahlen 3 erzeugt werden. Das Array weist eine Vielzahl von Array-Elementen auf, die jeweils in Gruppen zusammengefasst sind, wobei jede Gruppe einen bestimmten Schall-Frequenzbereich repräsentiert. So kann es zweckmäßig sein, unter Berücksichtigung einer realistischen Frequenzselektivität 25 Gruppen von Array-Elementen für die Erzeugung von 25 fokussierten Ultraschallstrahlen (z. B. mit identischer Ultraschallfrequenz) vorzusehen. Die Anzahl der Gruppen von Array-Elementen entspricht folglich der Anzahl der Frequenzgruppen in der Cochlea. Jede Gruppe ist folglich für eine bestimmte Region der Nervenfasern verantwortlich. Die Steuerungssingale erzeugen die Ultraschall-Strahlung 3 durch Anregung bestimmter Elemente bzw. Gruppen des Arrays. Die Dauer der Einstrahlung sowie die Wiederholung der erzeugten Stimulation/Blockierung wird für jedes einzelne Element oder für die Gruppe der Arrays durch die aus dem Sprachprozessor 5 codierten Steuerungssignale im Ultraschalltreiber 7 generiert. Die Kombination der jeweils aktiven Elemente/Gruppen wird nach der Einstrahlungsenergie des Ultraschalls 3 und der psychoakustischen Anforderungen der realistischen Hörwahrnehmung in Echtzeit erzeugt.

Dabei ist z. B. in Fig. 2 angedeutet, dass die einzelnen Ultraschallstrahlen 3 in verschiedene Bereiche der Cochlea H eingestrahlt werden. Es ist im Übrigen vorteilhaft, wenn mit der Ultraschallquelle 2 ein oder mehrere Referenzstrahlen 3' erzeugt werden, welche unabhängig von den im Schallwandler 1 erzeugten Signalen auf ein oder mehrere Referenzpunkte R fokussiert werden. Dieses ist in Fig. 1 angedeutet. Dort sind zwei Referenzstrahlen 3' erkennbar, die z. B. auf die Spitze der Cochlea H als Fixpunkt fokussiert werden. Sofern sich die Position des Hörgerätes bzw. der Ultraschalleinrichtung 2 während der Benutzung ändert, wird dieses mit Hilfe der Referenzstrahlen 3' detektiert und es kann in Echtzeit eine Korrektur der übrigen Ultraschallstrahlen 3 erfolgen. Die Position der Ultraschalleinrichtung 2 wird folglich während der Benutzung kontinuierlich durch ein oder auch zwei Referenzstrahlen 3' geprüft, indem die Spitze der Cochlea H abgetastet wird. Die Fokustiefen und die Einstrahlwinkel der übrigen Ultraschallstrahlen 3 werden dann relativ zu dem Referenzstrahl 3' in Echtzeit korrigiert.

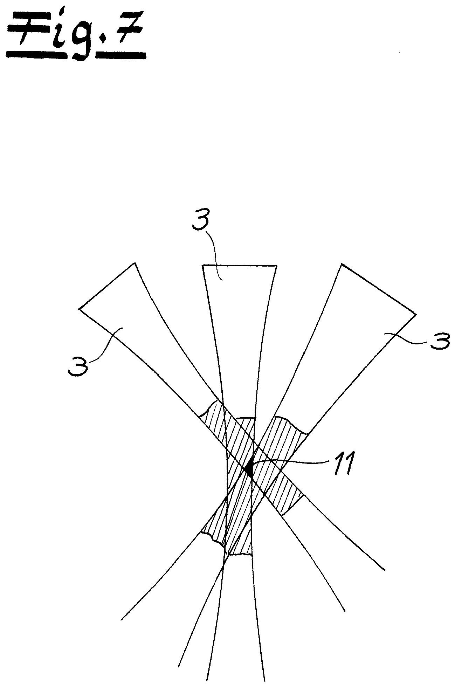

Im Übrigen ist in Fig. 1 angedeutet, dass zwei (oder auch mehr) mit Abstand zueinander erzeugte Ultraschallstrahlen 3 auf einen Punkt P fokussiert werden können, so dass dann durch Überlagerung der Strahlen ein reduziertes Fokusvolumen erzeugt wird. Dieses ist vergrößert in Fig. 7 am Beispiel von drei überlagerten, fokussierten Strahlen 3 dargestellt. Diese Überlegung trägt der Tatsache Rechnung, dass die einzelnen Fokusvolumina gegebenenfalls lateral schmal aber axial recht lang sein können. Durch Superposition von mehreren Ultraschallfeldern 3 kann ein Fokus 11 mit reduziertem Fokusvolumen erreicht werden (vgl. Fig. 7). Eine solche Vorgehensweise hat auch den Vorteil, dass die Belastung durch die Ultraschallstrahlung im Gewebe gering ist, da die erforderliche Stimulationsenergie erst im Fokus in der Cochlea erreicht wird.

Fig. 2 zeigt dabei eine erste Ausführungsform, bei der eine Exo-Hörprothese im Außenohr A positioniert wird. Diese Positionierung ist z. B. bei voller Taubheit des Patienten zweckmäßig.

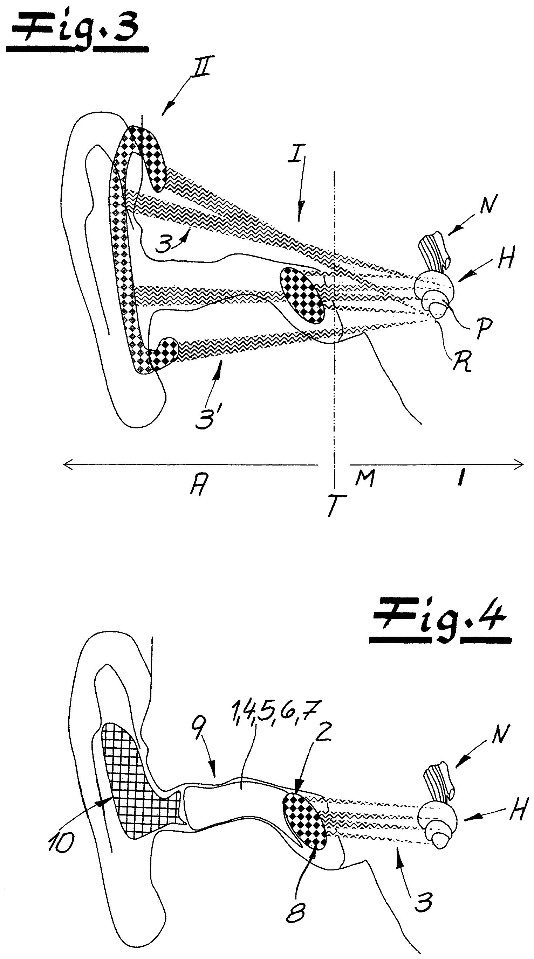

Demgegenüber zeigt Fig. 3 einerseits in Pos. I diese Ausführungsform nach Fig. 2 und zum Vergleich andererseits in Pos. II eine Ausführungsform mit einer Exo-Hörprothese, die außerhalb des Ohres befestigt wird. Diese Positionierung II kann z. B. bei Schwerhörigkeit oder auch bei Tinnitus zweckmäßig sein. Stets wird jedoch eine nichtinvasive Positionierung erreicht.

In Fig. 4 ist die Ausführungsform nach Fig. 2 dargestellt, jedoch zusätzlich mit einer elektronischen Steuereinheit 9 und einer Energieversorgungseinheit 10, die in dem dargestellten Ausführungsbeispiel optional von Solarzellen gebildet wird. Die elektronische Steuereinheit 9 weist das Mikrofon 1, den Analog/Digital-Wandler 4, den Sprachprozessor 5, den Codierer 6 und den Ultraschalltreiber 7 auf. Das Ultraschall-Array 8 ist ebenfalls angedeutet. Die Erfindung umfasst aber auch Ausführungsformen, bei denen andere Energieversorgungsmittel, z. B. Batterien, Akkumulatoren oder dergleichen vorgesehen sind.

Die Fig. 1 bis 4 zeigen dabei Ausführungsformen, bei denen eine Stimulation von Nervenzellen in der Cochlea H mit Ultraschallstrahlung erfolgt.

In Fig. 5 sind alternative Möglichkeiten der Stimulation angedeutet. Zunächst ist wieder der Bereich der Hörschnecke H erkennbar. Alternativ kann jedoch mit Hilfe der Ultraschallstrahlung auch eine Stimulation des Hörnervs N erfolgen, und zwar z. B. dann wenn die Hörschnecke H durch Krankheit derart beschädigt ist, dass eine Stimulation in der Hörschnecke H nicht mehr möglich ist. Sofern auch der Hörnerv N beschädigt oder aufgrund von Erkrankungen entfernt werden musste, besteht in einer Weiterentwicklung die Möglichkeit, andere Bereiche der Hörbahn im Gehirn G zu stimulieren, z. B. den Nucleus Cochlearis NC oder auch den Colliculus Inferior CI. Dabei macht sich die Erfindung die Tatsache zu Nutze, dass sich die Tonotopie (Frequenz-Orts-Abbildung) der Cochlea H bandförmig bis in das Gehirn fortsetzt. Es gibt folglich dort Bereiche, an denen jedem Ort eine bestimmte Frequenz zugeordnet ist, so dass die räumlich selektive Stimulation von Nervenzellen auch in diesen Bereichen des Gehirns möglich ist, z. B. dann, wenn eine Stimulation des Hörnervs nicht mehr möglich ist. Auch diese Umsetzung erfolgt jedoch nicht invasiv.

## Patentansprüche

1. Hörprothese, mit zumindest
- einem Schallwandler (1) zur Umwandlung akustischer Signale in elektrische Signale und
- einer Ultraschalleinrichtung (2), mit welcher eine Vielzahl von fokussierten, gepulsten Ultraschallstrahlen (3) erzeugbar sind,
wobei die Ultraschalleinrichtung (2) im Außenohr und/oder außerhalb des Ohres befestigbar ist und
wobei die Ultraschallstrahlen (3) in Abhängigkeit von den vom Schallwandler (1) erzeugten elektrischen Signalen auf verschiedene räumlich verteilte Punkte (P) in einem Bereich des Innenohrs (I) oder in einem Bereich der Hörbahn im Gehirn (G) unter Stimulierung von Nerven fokussierbar sind.

2. Hörprothese nach Anspruch 1, wobei die Ultraschalleinrichtung (2) zumindest ein Ultraschall-Array (8) aufweist, mit welchem die Vielzahl von (dynamisch) fokussierbaren Ultraschallstrahlen (3) erzeugbar sind.

3. Hörprothese nach Anspruch 1 oder 2, wobei mit der Ultraschalleinrichtung (2) mehr als 10, vorzugsweise mehr als 20 fokussierbare und ablenkbare Ultraschallstrahlen (3) erzeugbar sind.

4. Hörprothese nach einem der Ansprüche 1 bis 3, wobei die Pulslänge der Ultraschallpulse der Ultraschallstrahlen 0,01 ms bis 1 ms, vorzugsweise 0,05 ms bis 0,5 ms beträgt.

5. Hörprothese nach einem der Ansprüche 1 bis 4, wobei die Ultraschallfrequenz geringer als 1000 kHz, vorzugsweise geringer als 700 kHz, z. B. 100 bis 900 kHz, besonders bevorzugt 250 bis 700 kHz beträgt.

6. Hörprothese nach einem der Ansprüche 1 bis 5, wobei die Ultraschallpulse jeweils eine Pulsleistung von 0,01 bis 0,1 W/cm² aufweisen.

7. Hörprothese nach einem der Ansprüche 1 bis 6, wobei mit der Ultraschallquelle (2) ein oder mehrere Referenzstrahlen (3') erzeugbar sind, welche unabhängig von den mit dem Schallwandler (1) erzeugten Signalen auf ein oder mehrere Referenzpunkte (R) fokussierbar sind.

8. Verfahren zum Ausgleich von Hörverlusten oder Hörstörungen mit einer Hörprothese nach einem der Ansprüche 1 bis 7,
wobei die Hörprothese im Außenohr und/oder außerhalb des Ohres befestigt wird,
wobei die Ultraschallstrahlen (3) in Abhängigkeit von den vom Schallwandler (1) erzeugten elektrischen Signalen auf verschiedene räumlich verteilte Punkte (P) in einem Bereich des Innenohrs (I) oder in einem Bereich der Hörbahn im Gehirn (G) fokussiert werden und dadurch verschiedene räumlich verteilte Regionen in der Hörschnecke (H) oder in einem Bereich der Hörbahn, welche unterschiedliche Schallfrequenzen repräsentieren, stimulieren.

9. Verfahren nach Anspruch 8, wobei die einzelnen Ultraschallstrahlen (3) auf verschiedene räumlich verteilte Punkte (P) innerhalb der Hörschnecke (H) oder des Hörnervs (N) oder des Nucleus Cochlearis (NC) oder des Colliculus Inferior (CI) fokussiert werden.

10. Verfahren nach Anspruch 8 oder 9, wobei zur Erzeugung einer Vielzahl von dynamisch fokussierbaren Ultraschallstrahlen (3) ein Ultraschall-Array (8) verwendet wird, deren Array-Elemente in Gruppen aufgeteilt und zusammengefasst sind, welche jeweils einen akustischen Frequenzbereich repräsentieren.

11. Verfahren nach einem der Ansprüche 8 bis 10, wobei jeweils zumindest zwei mit Abstand zueinander erzeugte Ultraschallstrahlen (3) auf einen Punkt fokussiert und unter Erzeugung eines reduzierten Fokusvolumens überlagert werden.

12. Verfahren nach einem der Ansprüche 8 bis 11, wobei zumindest ein Referenzstrahl (3') unabhängig von den mit dem Schallwandler (1) erzeugten Signalen auf zumindest einen Referenzpunkt (R) fokussiert wird und wobei die Lage der Fokussierungen der Ultraschallstrahlen (3) in Abhängigkeit von dem Referenzstrahl (3') korrigiert werden, vorzugsweise dynamisch in Echtzeit.

## Claims

1. A hearing prosthesis, comprising at least
- an acoustic transducer (1) for converting acoustic signals into electrical signals, and
- an ultrasonic device (2) for generating a plurality of focused, pulsed ultrasonic beams (3),
wherein the ultrasonic device (2) can be attached to the outer ear and/or outside of the ear, and
wherein the ultrasonic beams (3) can be focused on various spatially distributed points (P) in an area of the inner ear (I) or in an area of the auditory pathway in the brain (G) with stimulation of nerves as a function of the electrical signals generated by the acoustic transducer (1).

2. The hearing prosthesis according to claim 1, wherein the ultrasonic device (2) has at least an ultrasonic array (8) with which the plurality of (dynamically) focusable ultrasonic beams (3) can be generated.

3. The hearing prosthesis according to claim 1 or 2, wherein more than ten, preferably more than twenty, focusable and deflectable ultrasonic beams (3) can be generated with the ultrasonic device (2).

4. The hearing prosthesis according to any one of claims 1 to 3, wherein the pulse length of the ultrasonic pulses of the ultrasonic beams amounts to 0.01 ms to 1 ms, preferably 0.05 ms to 0.5 ms.

5. The hearing prosthesis according to any one of claims 1 to 4, wherein the ultrasonic frequency is less than 1000 kHz, preferably less than 700 kHz, for example, 100 to 900 kHz, especially preferably 250 to 700 kHz.

6. The hearing prosthesis according to any one of claims 1 to 5, wherein the ultrasonic pulses each have a pulse power of 0.01 to 0.1 W/cm².

7. The hearing prosthesis according to any one of claims 1 to 6, wherein one or more reference beams (3'), which can be generated with the ultrasonic source (2), can be focused on one or more reference points (R), regardless of the signals generated by the acoustic transducer (1).

8. A method for compensating hearing loss or hearing disorders with a hearing prosthesis according to any one of claims 1 to 7,
wherein the hearing prosthesis is attached in the outer ear and/or outside of the ear,
wherein the ultrasonic beams (3) are focused at various spatially distributed points (P) in an area of the inner ear (I) or in an area of the auditory pathway in the brain (G) as a function of the electrical signals generated by the acoustic transducer (1) and therefore stimulate various spatially distributed regions in the cochlea(H) or in an area of the auditory pathway representing different sound frequencies.

9. The method according claim 8, wherein the individual ultrasonic beams (3) are focused on various spatially distributed points (P) within the cochlea (H) or the auditory nerve (N) or the cochlear nucleus (CN) or the inferior colliculus (IC).

10. The method according to claim 8 or 9, wherein an ultrasonic array (8) is used to generate a plurality of dynamically focusable ultrasonic beams (3), the array elements being divided into groups and combined into groups, each of which represents an acoustic frequency range.

11. The method according to any one of claims 8 to 10, wherein at least two ultrasonic beams (3) generated with a distance between them are focused at a point and are superimposed to create a reduced focal volume.

12. The method according to any one of claims 8 to 11, wherein at least one reference beam (3') is focused independently on at least one reference point (R), regardless of the signals generated by the acoustic transducer (1), and wherein the position of the focusing of the ultrasonic beams (3) is corrected as a function of the reference beam (3'), preferably being correctly dynamically in real time.

## Revendications

1. Prothèse auditive, comprenant au moins
- un transducteur acoustique (1) pour la conversion des signaux acoustiques en signaux électriques et
- un dispositif échographique (2) qui permet de générer une multitude de rayons ultrasoniques focalisés pulsés (3),
le dispositif échographique (2) pouvant être fixé dans l'oreille interne et/ou en dehors de l'oreille, et
les rayons ultrasoniques (3) pouvant être focalisés en fonction des signaux électriques générés par le transducteur acoustique (1) sur différents points répartis dans l'espace (P) dans une zone de l'oreille interne (I) ou dans une zone de la voie auditive dans le cerveau (G) sous la stimulation de nerfs.

2. Prothèse auditive selon la revendication 1, dans laquelle le dispositif échographique (2) présente au moins un réseau ultrasonique (8) grâce auquel la multitude de rayons ultrasoniques (3) pouvant être focalisés (dynamiquement) peuvent être générés.

3. Prothèse auditive selon une des revendications 1 ou 2, dans laquelle, grâce au dispositif échographique (2), plus de 10, de préférence plus de 20 rayons ultrasoniques (3) pouvant être focalisés et déviés peuvent être générés.

4. Prothèse auditive selon une des revendications 1 à 3, dans laquelle la longueur d'impulsion des impulsions ultrasoniques des rayons ultrasoniques s'élève à 0,01 ms à 1 ms, de préférence 0,05 ms à 0,5 ms.

5. Prothèse auditive selon une des revendications 1 à 4, dans laquelle la fréquence ultrasonique est inférieure à 1000 kHz, de préférence inférieure à 700 kHz, par exemple de 100 à 900 kHz, très préférentiellement de 250 à 700 kHz.

6. Prothèse auditive selon une des revendications 1 à 5, dans laquelle les impulsions ultrasoniques présentent respectivement une puissance d'impulsions de 0,01 à 0,1 W/cm².

7. Prothèse auditive selon la revendication 1 à 6, dans laquelle, grâce à la source ultrasonique (2), peuvent être générés un ou plusieurs rayons de référence (3') qui, indépendamment des signaux générés avec le transducteur acoustique (1), peuvent être focalisés sur un ou plusieurs points de référence (R).

8. Procédé de compensation des pertes auditives ou des troubles auditifs à l'aide d'une prothèse auditive selon une des revendications 1 à 7,
dans lequel la prothèse auditive est fixée dans l'oreille externe et/ou en dehors de l'oreille,
les rayons ultrasoniques (3) sont focalisés, en fonction des signaux électriques générés par le transducteur acoustique (1), sur différents points répartis dans l'espace (P) ou dans une zone de l'oreille interne (I) ou dans une zone de la voie auditive dans le cerveau (G) et stimulent ainsi différentes zones réparties dans l'espace dans la cochlée (H) ou dans une zone de la voie auditive qui représente différentes fréquences sonores.

9. Procédé selon la revendication 8, dans lequel les rayons ultrasoniques individuels (3) sont focalisés sur différents points répartis dans l'espace (P) dans la cochlée (H) ou dans le nerf auditif (N) ou le noyau cochléaire (NC) ou le colliculus inférieur (CI).

10. Procédé selon la revendication 8 ou 9, dans lequel, pour générer une multitude de rayons ultrasoniques pouvant être focalisés dynamiquement (3), est utilisé un réseau ultrasonique (8) dont les éléments de réseau sont divisés en groupes et réunis et qui représentent respectivement une plage de fréquence acoustique.

11. Procédé selon une des revendications 8 à 10, dans lequel respectivement au moins deux rayons ultrasoniques générés à distance l'un de l'autre (3) sont focalisés sur un point et superposés en générant un volume focal réduit.

12. Procédé selon une des revendications 8 à 11, dans lequel au moins un rayon de référence (3') est focalisé indépendamment des signaux générés à l'aide du transducteur acoustique (1) sur au moins un point de référence (R) et la position des focalisations des rayons ultrasoniques (3) est corrigée en fonction du rayon de référence (3'), de préférence dynamiquement en temps réel.
